# EUROPÄISCHE PATENTSCHRIFT

(11) **EP 0 969 918 B1**
(45) Veröffentlichungstag und Bekanntmachung des Hinweises auf die Patenterteilung: **08.01.2003**
(21) Anmeldenummer: 98909410.7
(22) Anmeldetag: 07.02.1998
(51) Int. Cl.: B01J 19/00, C07B 61/00

(54) **VERFAHREN ZUR HERSTELLUNG VON STRUKTURIERTEN, SELBSTORGANISIERTEN MOLEKULAREN MONOLAGEN EINZELNER MOLEKULARER SPEZIES, INSBESONDERE VON SUBSTANZBIBLIOTHEKEN**
METHOD FOR PRODUCING STRUCTURED, SELF-ORGANIZED MOLECULAR MONOLAYERS OF INDIVIDUAL MOLECULAR SPECIES
PROCEDE DE PRODUCTION DE MONOCOUCHES MOLECULAIRES STRUCTUREES AUTO-ORGANISEES D'ESPECES MOLECULAIRES INDIVIDUELLES, EN PARTICULIER DE BIBLIOTHEQUES DE SUBSTANCES

(30) Priorität: 19.02.1997 DE 19706570
(43) Veröffentlichungstag der Anmeldung: 12.01.2000
(73) Patentinhaber: Clondiag Chip Technologies GmbH, 07743 Jena (DE)
(72) Erfinder: ERMANTRAUT, Eugen, D-07745 Jena (DE); KÖHLER, Johann, Michael, D-07751 Golmsdorf (DE); SCHULZ, Torsten, D-07743 Jena (DE); WOHLFART, Klaus, D-07751 Laasan (DE); WÖLFL, Stefan, D-83059 Kolbermoor (DE)
(74) Vertreter: Pfeiffer, Rolf-Gerd, Dipl.-Phys.
(86) Internationale Anmeldenummer: EP9800676
(87) Internationale Veröffentlichungsnummer: WO98036827

(56) Entgegenhaltungen:
- WO-A-97/06468
- WO-A-97/33737
- US-A- 5 258 091
- US-A- 5 429 807
- US-A- 5 489 678
- US-A- 5 599 695

## Beschreibung

Die Erfindung betrifft ein Verfahren zur Herstellung von strukturierten, selbstorganisierten molekularen Monolagen einzelner molekularer Spezies. Bevorzugt findet die Erfindung Anwendung zur Ausbildung von festphasengekoppelten Substanzbibliotheken oder von anderen Assays zur Testung molekularer Wechselwirkungen.

Derartige Bibliotheken, die dem schnellen Auffinden von wechselwirkenden Partnern auf molekularer Ebene dienen, sind grundsätzlich bekannt. Solche Substanzbibliotheken werden nach dem bekannten, der Erfindung am nächsten kommenden Stand der Technik auf verschiedene Weise erzeugt. So ist der Einsatz sogenannter "microbeads", worunter kleine Perlen zu verstehen sind, die an ihrer Oberfläche einen molekularen Reaktionspartner tragen, bekannt. Weiterhin ist zur Herstellung solcher Bibliotheken ein sogenanntes "mix&split"-Verfahren [Erb, E.; Jander, K.D.; Brenner, S.: Recursive Deconvolution of Combinatorial Chemical Libraries, Proc. of Natl. Acad. Sci. USA 91, S. 11422-6 (1994)] bekannt.
Besondere Vorteile bieten festphasengekoppelte Bibliotheken, deren Komponenten eindeutig durch ihre Position (x-y-Richtung) bestimmt sind. Zur seriellen Erzeugung solcher Bibliotheken werden z.B. modifizierte Strahldrucker eingesetzt, die die miteinander in Verbindung zu bringenden Substanzen auf ein vorgegebenes Substrat punktförmig aufsprühen (Schober, A. Günther, R.; Schwienhorst, A. Döring, M.; Lindemann, B.F.: Accurate high-speed liquid Handling of very small biological samples, Biotechniques 15: S. 324-9, 1993). Zur parallelen Erzeugung solcher Bibliotheken sollen sogenannte "applicator" Verwendung finden, bei denen ein Kopf, versehen mit vielen dünnen Kanälen, auf eine Oberfläche aufgesetzt wird und zugleich eine Vielzahl von Mikroreaktionsräumen bedienen kann; vgl. z.B.US 5,429,807. Für genannte Zwecke ist weiterhin der Einsatz von lichtaktivierbaren Schutzgruppen (US 5,489,678) und von Stempeltechniken (DE 195 43 232.0) bereits beschrieben.
Diesen bekannten Verfahren und Vorrichtungen haften ein oder mehrere prinzipielle Nachteile an:
So kann durch den Einsatz von Strahldruckern nicht gleichzeitig eine größere Anzahl von Oberflächenelementen auf einmal mit einer Substanz bedient werden, da es sich dabei um einen seriellen Prozeß handelt. Die als "applicator" bekannten Vorrichtungen stoßen bzgl. ihrer Miniaturisierung an technisch mögliche Grenzen. Stempelsynthesen, die sich oberflächenstrukturierter und -profilierter Stempel zur Übertragung einer Substanz bedienen, verursachen erhebliche Probleme bei der exakten Positionierung; reproduzierbare Mehrfachsynthesen sind damit praktisch ausgeschlossen.
Bekannte Verfahren der komplexen Schutzgruppenchemie unter Einsatz lichtaktivierbarer Schutzgruppen erfordern lange Belichtungszeiten, wenn eine hohe Schritteffizienz angestrebt wird, wodurch der Gesamtprozeß zur Herstellung einer Bibliothek sehr zeitaufwendig wird. Außerdem bedingt die Lichtempfindlichkeit letzt genannter Verfahren eine besonders aufwendige Prozeßdurchführung. In Proc. Natl. Acad. Sci. USA, Vol. 93, Nov. 96, S. 13555-13560 ist ein derartiges Verfahren zur Erzeugung von Substanzbibliotheken beschrieben, bei dem ein Substrat zunächst mit einer geeigneten Schutzgruppe oder mit einem eine erste Schutzgruppe tragenden Monomer versehen wird. Danach wird eine Substratoberfläche mit einem photoaktivierbaren Lackresist beschichtet, Teilbereiche entsprechend einem vorgegebenen Synthesealgorithmus belichtet und strukturiert. In den strukturierten Bereichen erfolgt die Abspaltung der Schutzgruppe, in den freigelegten Bereichen kann danach die Anbindung eines zweiten Monomers erfolgen. Nach Entfernung der übrigen Lachschichten kann der gesamte Prozeß mit einer entsprechend angepaßten, neu zu strukturierenden Lackschicht wiederholt werden. Der Hauptnachteil neben oben bereits genannten Nachteilen dieser Vorgehensweise besteht in der nicht vollständigen Entfernung von Fotolackresten von der Bibliotheksmatrix, wodurch die Ausbeute pro Syntheseschritt und damit die Gesamtsyntheseausbeute sinkt.

Der Erfindung liegt die Aufgabe zugrunde, ein Verfahren zur Herstellung von strukturierten, selbstorganisierten molekularen Monolagen einzelner molekularer Spezies anzugeben, das reproduzierbare und mehrfache Wiederholungen der Bildung und lokal definierten Anbindung von molekularen Bausteinen an molekulare Monolagen mit einer hohen Ausbeute zuläßt. Das zu schaffende Verfahren soll insbesondere die Ausbildung von Testassays und festphasengebundenen Substanzbibliotheken ermöglichen.
Die Aufgabe wird durch die kennzeichnenden Merkmale der Patentansprüche gelöst.

Gemäß der Erfindung gelangen speziell präparierte, mikrostrukturierte Polymermasken zum Einsatz, die für den Andruck an ein angepaßtes Substrat ausgelegt sind und welche in vorgegebenen Bereichen Öffnungen aufweisen, die die Erzeugung von monomolekularen Monolagen auf dem Substrat ermöglichen.

Die Erfindung soll nachstehend anhand schematischer Ausführungsbeispiele näher erläutert werden. Es zeigen:
- Fig. 1a bis 1e: eine Ausbildungsmöglichkeit für die Herstellung der erforderlichen Polymermasken,
- Fig. 2: ein schematisches Ablaufschema zur Herstellung einer festphasengekoppelten Substanzbibliothek anhand eines speziellen Beispiels,
- Fig. 3: ein Beispiel für eine nach der Erfindung in z-Richtung nanostrukturierte Oberfläche,
- Fig. 4: andeutungsweise einen Teil eines Satzes unterschiedlichen Polymermasken zur Erzeugung von Substanzbibliotheken.

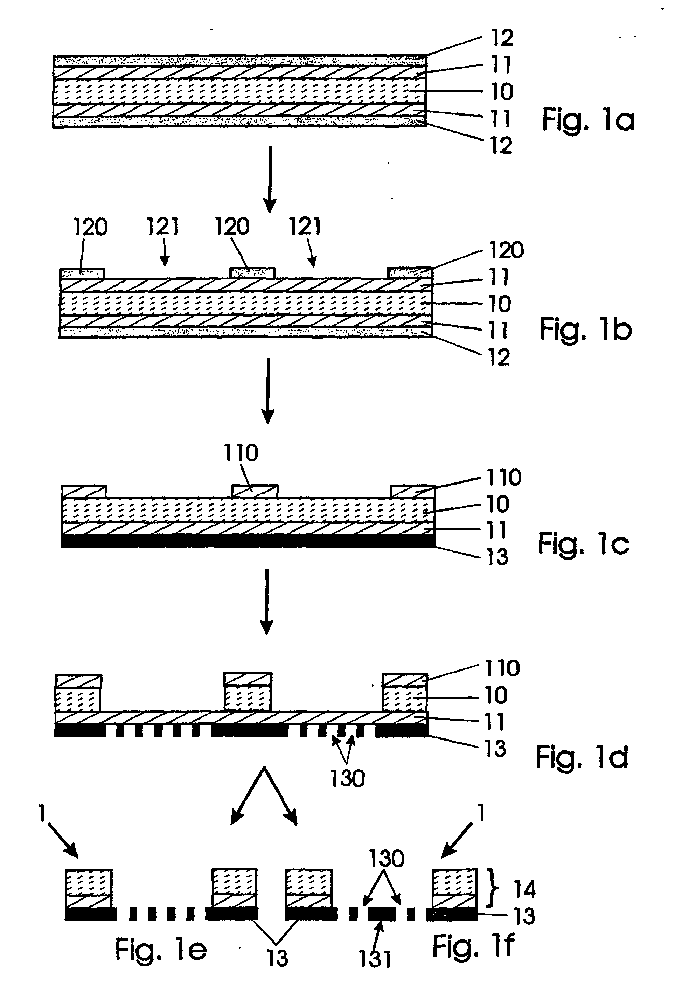

In den Figuren 1a bis 1d ist eine beispielhafte Ausbildungsmöglichkeit für die im Rahmen der Erfindung erforderlichen Polymermasken dargestellt. Ausgangspunkt ist dabei ein in Fig. 1a dargestellter 4"-Siliziumwafer 10, der beidseitig mit einer thermischen SiO₂-Oxidschicht 11 einer Dicke von 500 nm beschichtet ist. Die SiO₂-Oxidschichten 11 sind mit einer, mittels spin-coating aufgebrachter, in der Mikrostrukturierung üblicher Photoresistschicht 12 abgedeckt. Eine dieser Photoresistschichten 12 wird mit einer nicht dargestellten Maske versehen, die so ausgeführt ist, daß im weiter zu beschreibenden Prozeßablauf ein an das später eingesetzte Substrat 2 in seinen Abmaßen angepaßter Rahmen 14 nach erfolgter vollständiger Polymermaskenstrukturierung verbleibt. Die diesbezüglich verbleibenden Photoresistrestschichtbereiche 120 sind in Fig. 1b gezeigt, wodurch Fenster 121 freigelegt werden. In die erste SiO₂-Oxidschicht 11 werden entsprechend der Maskenvorgabe Fenster eingeätzt und die Fotoresistschichten entfernt; das Ergebnis dieser Schritte, mit verbleibenden SiO₂-Maskenbereichen 110, ist in Fig. 1c dargestellt. Weiterhin zeigt Fig. 1c eine neu aufgebrachte photostrukturierbare Polymerschicht 13, die im Beispiel aus PDMS (Polydimethylsiloxan) unter Zusatz eines Quervernetzers, wie z.B. eine in organischen Lösungsmitteln, wie z.B. Xylol oder Chloroform, lösliche Diazidoverbindung oder 2,2-Dimethoxy-2-phenyl-acetophenon, gefertigt ist. Es liegt im Rahmen der Erfindung, je nach späteren Einsatzfällen und verwendeten Agenzien für diese Polymerschicht 13 auch andere Materialien einzusetzen, bspw. Polyvinylalkohol, PMMA, Polyvinylpyrollidon usw., die mit einem geeigneten Vernetzer versehen wurden, wie z.B. einem Salz des Diazidostilbensulfats. Diese Polymerschicht 13 wird mit einer weiteren, nicht dargestellten Maske versehen, deren Struktur denen später auf ein noch zu beschreibendes Substrat 2 durch die zu erzeugenden Strukturen in der Polymerschicht 13 zu übertragenden Strukturen entspricht. Figur 1d zeigt das Ergebnis der Strukturerzeugung in der Polymerschicht 13 mit erzeugten Maskenfenstern 130. Zugleich ist in Fig. 1d dargestellt, wie die Siliziumbereiche, die nicht von den SiO₂-Maskenbereichen 110 abdeckt wurden, in einem weiteren Prozeßschritt unter Einsatz bekannter Ätzmittel entfernt wurden. Anschließend werden in einem weiteren Ätzbad alle nicht abgedeckten SiO₂-Bereiche entfernt. Das Ergebnis eines einzelnen Siliziumrahmens, der von einer freitragenden, strukturierten Polymermaske 13 überspannt ist, ist in Fig. 1e dargestellt. Diese im weiteren als mikrostrukturierte Polymermaske 1 bezeichnete Baugruppe findet im Rahmen der Erfindung ein bis mehrfach Anwendung. Zur Herstellung von Substanzbibliotheken werden weitere solcher Polymermasken 1 benötigt, die im weiteren alle mit 1 bezeichnet sind, deren vorgegebene Öffnungsfenster 130 grundsätzlich zueinander deckungsgleich ausgeführt, jedoch teilweise, an einen Synthese- bzw. Modifikationsalgorithmus angepaßt, uneröffnet ausgeführt sind, wie es in Fig. 1f durch einen Pfeil 131 schematisch angedeutet ist. Die Art der Strukturerzeugung in genannten Polymerfilmen 13 ist im Rahmen der Erfindung unerheblich; so können auch sogenannte Trockenätzverfahren zum Einsatz gelangen, wenn entsprechend kleine Öffnungen bis in den Submikrometerbereich erforderlich sind. Wesentlich ist lediglich, daß die erzeugte Polymermaske so dünn und flexibel gefertigt ist, daß sie auf ein im weiteren zu beschreibendes Substrat 2 aufgelegt, bereits durch Adhäsion haftet und sie gegen die im weiteren Prozeß zum Einsatz gelangenden Agenzien chemisch stabil ist. Ohne dies näher darzustellen, sind sämtlichen im Prozeß eingesetzter Polymermasken 1 und den zum Einsatz gelangenden Substraten 2 entsprechende Justiermarken gegeben, die eine exakte Ausrichtung z.B. mit Hilfe in der Mikrostrukturierung üblicher Maskaligner ermöglichen.

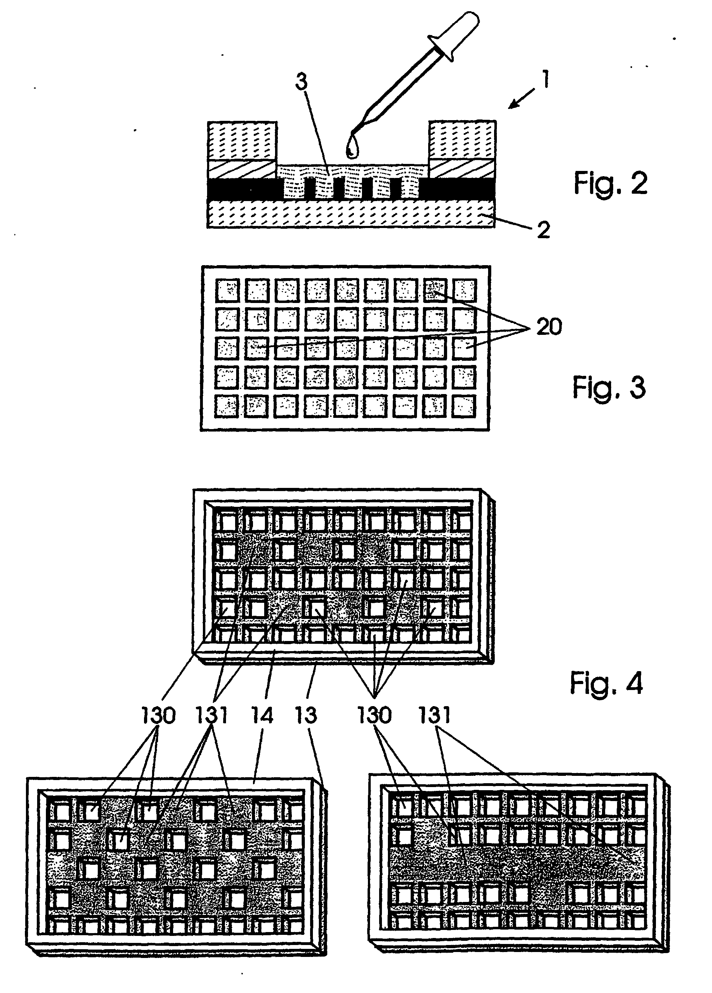

Im weiteren soll beispielhaft anhand von Fig. 2 die Präparation eines Substrates 2 im Sinne der Erfindung beschrieben werden. Als Substrat 2 soll ein mit Gold beschichteter Siliziumwafer Anwendung finden. Dieser soll frisch präpariert sein und kann in doppelt destilliertem Wasser gelagert werden. Auf das Substrat 2 wird eine nach vorherigem Beispiel hergestellte Polymermaske 1 aufgelegt und mit einer Reaktionslösung 3, mit der die im übrigen nicht näher dargestellte Goldoberfläche reagieren soll, überschwemmt. Im Beispiel wird für die Reaktionslösung 3 NHS-SS-Biotin gelöst zu 10 µg/ml in Phosphatpuffer, 0,01 M, pH 8,5 gewählt. Nach ca. 15 min Inkubation bei Raumtemperatur wird die Oberfläche mit reinem Puffer und destilliertem Wasser gewaschen und die Polymermaske 1 abgezogen. Daran anschließend wird über die gesamte Oberfläche des Substrats 2 Streptavidin gegeben, welches mit einer Affinität von ca. 10⁻¹⁵ M an in den durch die Öffnungsbereiche 130 der Polymermaske 1 vorgegebenen Regionen immobilisierte Biotin bindet. Damit sind definierte Bereiche 20 auf der Goldoberfläche geschaffen, von denen in Fig. 3 nur drei mit Bezugslinien versehen sind, welche ausschließlich mit Streptavidin besetzt sind. Eine solcherart hergestellte monomolekulare Monolage, die in mehrere, regelmäßig verteilte Bereiche 20 unterteilt ist, läßt sich bspw. als Immobilisierungsmatrix für biotinylierte Moleküle verwenden, aber auch als Substrat mit räumlich selektiver Adhäsion oder definierter Grenzflächenenergie einsetzen.

In einem weiteren Ausführungsbeispiel soll die Präparation eines Substrats beschrieben werden, das als Ausgangssubstrat für die Ausbildung einer Substanzbibliothek dienen soll. Als Substrat 2 wird hier bspw. ein gereinigtes Siliziumplättchen eingesetzt, dessen gesamte Oberfläche im Beispiel zunächst durch eine Behandlung mit 3-Glycidoxy-propyl-trimethoxisilan modifiziert werden soll. Das 3-Glycid-oxypropyl-trimethoxysilan (10% in Toluol, 80°C, ca. 8h), wird über das Substrat 2 geschwemmt. Eine zweistündige Inkubation des Substrates in 0,05 M HCl führt zur Hydrolyse des genannten Epoxids, es liegen damit freie Hydroxygruppen vor. Diese reagieren bspw. spontan mit Phosphoramiditen. Dazu wird im Beispiel ein C18-Spacer-Phosphoramidit aktiviert mit Tetrazol in 0,1 M Lösung in Acetonitril mit der modifizierten Substratoberfläche für 10 min zur Reaktion gebracht. Danach wird die Oberfläche gründlich mit Acetonitril gewaschen. Die Oberfläche des Substrats ist jetzt mit Dimethoxytritylgruppen besetzt, die ihrerseits bekannte Standardschutzgruppen in der Phosphoramitidschutzgruppenchemie sind.
Auf das so präparierte Substrat 2 wird, analog wie zu Fig. 2 beschrieben, eine bspw. nach Fig. 1 gefertigte Polymermaske 1 aufgelegt und mittels eines Maskaligners entsprechend positioniert. Nach erfolgter Positionierung kann das Sandwich, bestehend aus dem Substrat 2 und der Polymermaske 1 aus der Halterung entnommen werden, da die Polymermaske, aufgrund ihrer Ausführung, adhäsiv auf der Oberfläche des Substrats haftet. Über diesen Sandwichverbund wird 2% Triflouressigsäure in H₂O gegeben, wobei in den Polymermaskenöffnungsbereichen 130 eine Abspaltung der nach obigem Beispiel erzeugter Schutzgruppen stattfindet. Danach wird die Polymermaske 1 vom Substrat abgezogen und es liegen analog zu Fig. 3 verteilte, von einer vordefiniert auswählbaren Schutzgruppe befreite Bereiche 20 vor. Bereits in diesem Zustand kann das Substrat als Testassay Verwendung finden. Die jeweils spezielle Art der Substratpräparation ist mannigfaltig und an eine Vielzahl späterer Verwendungen anpaßbar.
Dieses so vorbereitete Substrat kann im weiteren bspw. einem an sich bekannten Phosphoramiditzyklus unterworfen werden, um ein weiteres Oligonukleotidmonomer an die bezeichneten Bereiche 20 anzusynthetisieren.
Zum Aufbau einer Substanzbibliothek werden im Rahmen der Erfindung weitere Polymermasken 1 vorgesehen, die entsprechend der zu synthetisierenden Stoffgruppe variabel anpaßbar derart ausgestaltet sind, daß ihre Öffnungen 130 grundsätzlich mit den Öffnungen 130 der ersten Polymermaske deckungsgleich korrespondieren, jedoch teilweise uneröffnete Maskenbereiche 131 aufweisen, wie sie beispielhaft in Fig. 4 angedeutet sind. Ebenso liegt es im Rahmen der Erfindung, bei Bedarf größere Öffnungen auszubilden und damit größere benachbarte Bereiche auf dem Substrat anzusprechen. Zur Verlängerung der molekularen Spezies an den vorgesehenen Orten wird eine dem Synthesezyklus entsprechende neue Polymermaske aufgelegt, die entsprechend zu synthetisierende Substanz aufgegeben, entsprechende Reaktions- und Reinigungsschritte durch geführt und anschließend die verwendete Polymermaske wieder abgezogen. Trotz der dadurch bedingten unterschiedlichen Wachstumshöhe einzelner molekularer Spezies, bestehend aus jeweils mehreren Monomeren, in den verschiedenen Synthesebereichen des Substrats bleibt die adhäsive Haftung der jeweiligen Polymermasken erhalten und es kommt selbst bei synthetisierten Schichtpaketen mit Kettenlängen von 30 - 100 oder mehr, nicht zu einer Unterspülung der jeweiligen Polymermasken durch die nachfolgende Syntheselösung. Auf die beschrieben Weise sind, unter Zuhilfenahme apparativer Technik aus dem Bereich der Mikrostrukturierung, zuverlässig Substanzbibliotheken mit hoher Reproduzierbarkeit und Ausbeute aufbaubar.
Der Aufbau beliebiger Polymer- und Substanzbibliotheken, wie z.B. Peptid-, Polysacharid-, Polyterpen- usw. Bibliotheken ist mit Hilfe der Erfindung durchführbar. Es ist auch die Herstellung gemischter Bibliotheken, worunter das Aufbringen von molekularen Spezies unterschiedlicher Substanzklassen auf einem Substrat verstanden werden soll, möglich. Ebenso besteht die Möglichkeit des Aufbaus von Bibliotheken mit Mischpolymeren, worunter Polymere, bestehend aus Monomeren unterschiedlicher Substanzklassen, verstanden werden. Vorausetzung hierfür ist die Kompatibilität der Schutzgruppen bzw. der funktionellen Kopplungsgruppen. Die Herstellung von verzweigten Polymeren bei Verwendung entsprechender mehrfach funktionalisierter, mehrfach geschützter Monomere ist ebenso möglich.
Die beschrieben Probleme nach dem Stand der Technik hergestellter Substanzbibliotheken werden wirkungsvoll vermieden. Die in den verwendeten Polymermasken vorgesehenen Öffnungen sind bis in den Submikrometerbereich herstellbar. Das Verfahren bietet den grundsätzlichen Vorteil, keinerlei Restriktion an die jeweils angewandte Chemie zu stellen. Die Materialien der verwendeten mechanischen Teile sind grundsätzlich dem Reaktionszyklus anpaßbar.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1 -: Polymermaske
- 10 -: Siliziumwafer
- 11 -: SiO₂-Oxidschicht
- 110 -: SiO₂-Maskenbereiche
- 12 -: Photoresistschicht
- 120 -: Photoresistrestschichtbereiche
- 121 -: Fenster
- 13 -: Polymerschicht
- 130 -: Öffnungsbereiche in der Polymerschicht 13
- 131 -: uneröffhete Maskenbereiche in der Polymerschicht 13
- 14 -: Rahmen
- 2 -: Substrat
- 20 -: definierte Bereiche auf dem Substrat 2

## Patentansprüche

1. Verfahren zur Herstellung von strukturierten, selbstorganisierten molekularen Monolagen einzelner molekularer Spezies, insbesondere von Substanzbibliotheken, **dadurch gekennzeichnet, daß**
a) auf ein, dem vorgesehenen Verwendungszweck und einer herzustellenden ersten Monolage angepaßt ausgewähltem oder speziell präpariertem Substrat
b) eine mikrostrukturierte Polymermaske, die definiert vorgegebene, jeweils gewünscht anzusprechenden Bereichen des Substrats entsprechende Öffnungen aufweist, wenigstens einmal auf das Substrat definiert ausgerichtet aufgelegt wird,
wobei die Polymermaske so dünn und flexibel ausgeführt ist, daß sie bereits durch Adhäsion auf dem Substrat haftet und
die Auswahl des für die Polymermaske eingesetzten Materials nach Maßgabe weiterer im jeweiligen Prozeßschritt zum Einsatz gelangender Agenzien derart erfolgt, daß das Polymermaskenmaterial gegen diese chemisch stabil ist,
c) das Sandwich, bestehend aus Substrat und Polymermaske, mit einem dem Anwendungszweck entsprechenden ersten Agens, zur Bildung wenigstens einer ersten molekularen Monolage in den Maskenöffnungsbereichen, überschwemmt wird,
c1) der Schritt nach c) je nach auszubildender Monolagenstruktur mit dem ersten Agens oder ein oder mehreren weiteren Agenzien wiederholt wird und
d) nach Ausführung des Schritts nach c) oder der Schritte nach c) und c1) die Polymermaske nach erfolgter, den Maskenöffnungsbereichen entsprechenden Modifikation der, dem Agens oder den Agenzien ausgesetzten Substratoberflächenbereiche abgezogen wird.

2. Verfahren nach Anspruch 1, **dadurch gekennzeichnet, daß** die Schritte nach b) bis d) jeweils unter Verwendung weiterer Polymermasken und weiterer Agenzien derart mehrfach wiederholt werden, daß die weiteren Polymermasken eine deckungsgleich justierte Ausrichtung zur Ausrichtung der ersten Polymermaske erfahren, die in den weiteren Polymermasken vorgesehenen Öffnungen grundsätzlich mit den Öffnungen der ersten Polymermaske deckungsgleich korrespondierend festgelegt sind, jedoch teilweise, dem jeweiligen Synthese- oder Modifikationsalgorithmus angepaßt, uneröffnet ausgeführt werden.

3. Verfahren nach Anspruch 1 und 2, **dadurch gekennzeichnet, daß** Polymermasken eingesetzt werden, die aus einem Rahmen gebildet sind, dessen äußere Abmaße im wesentlichen den äußeren Abmessungen des Substrats, auf das sie aufgelegt werden, entsprechen und der Rahmen substratseitig von einer Polymerfolie überspannt ist, in die genannte Öffnungen eingebracht sind.

## Claims

1. Method for producing structured, self-organizing molecular monolayers of individual molecular species, in particular, substance libraries, **characterized in that**
a) on a selected or specially prepared substrate adapted to the intended application and to a first monolayer to be produced
b)a microstructurized polymeric mask is deposited, at least once definedly aligned relative to said substrate, said mask being provided with definedly preselected openings corresponding to areas of said substrate to be addressed at will each,
wherein the polymeric mask is designed thin and flexibly enough that it sticks to the substrate only by adhesion, and **in that**
the selection of the material employed for the polymeric mask is in accordance with further agents used in the respective step of proceeding in such a manner that the material of the polymeric mask is chemically stable against said agents,
c) the sandwich, constituted of said substrate and said polymeric mask, is flooded by a first agent, adapted to the purpose of application, for the formation of at least a first molecular monolayer in the opening areas of the mask,
c1) the step according to c) is repeated depending on the monolayer structure to be formed with the first agent or one further agent or a plurality of further agents, and
d)the polymeric mask is stripped off after completion of the step according to c) or the steps according to c) and c1) subsequent to the completed modification, corresponding to the opening areas of the mask, of the surface ranges of the substrate having been subjected to the agent and the agents, respectively.

2. Method according to claim 1, **characterized in that** the steps according to b) to d) can be multiply repeated under use of further polymeric masks and further agents in such a manner that the further polymeric masks are adjusted in a congruent alignment relative to the alignment of said first polymeric mask, the openings provided in said further polymeric masks are stipulated always in congruent analogy to the openings of said first polymeric mask, being, however, partially executed non-opened, in order to match the respective synthesis or modification algorithm.

3. Method according to claims 1 and 2, **characterized in that** polymeric masks are employed constituted of a frame, the external dimensions of which substantially correspond to the external dimensions of the substrate upon which they are deposited, and **in that** the frame is overlapped, on the side of the substrate, by a polymeric film, into which said openings are inserted.

## Revendications

1. Le procédé permettant la préparation de monocouches moléculaires structurées et auto-organisés de certaines espèces moléculaires, notamment de bibliothèques de substances, est caractérisé en ce
a) que sur le substrat spécialement préparé ou sélectionné en fonction de la première monocouche à préparer et de l'usage prévu,
b) est appliqué au moins une fois et dans un sens bien défini un masque de polymère microstructuré présentant des ouvertures prédéfinies et orientées en fonction des zones à solliciter du substrat,
le masque de polymère étant exécuté de manière si mince et si souple qu'il se colle par simple adhérence contre le substrat,
la sélection des matières utilisées pour la préparation du masque de polymère étant effectuée en tenant compte des agents appliqués dans l'étape concerné du processus de sorte, qu'elles présentent la stabilité chimique nécessaire en contact avec ces agents,
c) que le sandwich composé du substrat et du masque de polymère, est submergé d'un premier agent répondant aux besoins d'utilisation, pour former au moins une première monocouche moléculaire dans les zones d'ouverture du masque ;
c1) que suivant la structure à former de la monocouche, l'opération selon c) sera répétée avec le même ou avec un on plusieurs d'autres agents et
d) que, après achèvement de l'opération suivant c) ou des opérations suivant c) et c1), le masque de polymère sera enlevé après avoir obtenu la modification recherchée des zones surfaciques du substrat sur lesquels l'agent ou les agents ont pu agir à travers les ouvertures dans le masque de polymère.

2. Le procédé suivant la revendication 1 est **caractérisé en ce que** les opérations suivant b) à d) puissent être répétées à plusieurs reprises avec la mise en oeuvre d'autres masques de polymère et d'autres agents de sorte que les autres masques de polymère soient ajustés de manière égale par rapport au premier masque de polymère, que les ouvertures sur les autres masques de polymère, en principe, correspondent également aux ouvertures du premier masque de polymère, mais dont quelques unes demeurent fermées en fonction de l'algorithme de synthèse ou de l'algorithme de modification concerné.

3. Le procédé suivant les revendications 1 et 2 est **caractérisé en ce que** les masques de polymère mis en oeuvre sont composés d'un cadre dont les dimensions extérieures correspondent dans l'essentiel aux dimensions du substrat sur lequel ils seront appliqués, et que, côté substrat, le cadre est couvert d'un film en polymère, dans lequel les ouvertures indiquées sont aménagées.
